# FASCICULE DE BREVET EUROPEEN

(11) **EP 2 675 342 B1**
(45) Date de publication et mention de la délivrance du brevet: **03.07.2019**
(21) Numéro de dépôt: 12703553.3
(22) Date de dépôt: 14.02.2012
(51) Int. Cl.: A61B 5/00, A61M 16/00, G16H 40/67

(54) **BORNE D'ASSISTANCE PERMETTANT LA SURVEILLANCE A DISTANCE D'UNE PERSONNE CONNECTEE A UN DISPOSITIF MEDICAL D'ASSISTANCE ET DE SURVEILLANCE**
ENDGERÄT ZUR UNTERSTÜTZUNG BEI DER FERNÜBERWACHUNG EINER MIT EINER MEDIZINISCHEN HILFSVORRICHTUNG VERBUNDENEN PERSON UND ÜBERWACHUNGSVORRICHTUNG DAFÜR
ASSISTANCE TERMINAL FOR REMOTELY MONITORING A PERSON CONNECTED TO A MEDICAL ASSISTANCE AND MONITORING DEVICE

(30) Priorité: 14.02.2011 FR 1151192
(43) Date de publication de la demande: 25.12.2013
(73) Titulaire: Université Pierre et Marie Curie (Paris 6), 75005 Paris (FR)
(72) Inventeur: SIMILOWSKI, Thomas, F-92130 Issy-les-moulineaux (FR); GONZALEZ-BERMEJO, Jésus, F-95680 Montlignon (FR); STRAUS, Christian, F-78280 Guyancourt (FR); HURBAULT, Julien, F-75011 Paris (FR); FORET, Didier, F-75013 Paris (FR); FRANCKHAUSER, Nathalie, F-94700 Maisons Alfort (FR); ROUAULT, Sylvie, F-92290 Chatenay-malabry (FR)
(74) Mandataire: Parzy, Benjamin Alain
(86) Numéro de dépôt international: PCT/EP2012/052501
(87) Numéro de publication internationale: WO 2012/110504

(56) Documents cités:
- WO-A2-2009/153535
- US-A- 5 651 070
- US-A1- 2004 155 770
- US-A1- 2006 017 558

## Description

Borne d'assistance permettant la surveillance à distance d'une personne connectée à un dispositif médical d'assistance et de surveillance.

La présente invention concerne une borne d'assistance permettant de solliciter l'intervention d'une personne à surveiller et/ou d'un surveillant distant en cas de dégradation de l'état clinique de la personne à surveiller ou d'un disfonctionnement d'un dispositif médical d'assistance et de surveillance auquel est associée la borne d'assistance. Le surveillant distant peut tout aussi bien être un professionnel de santé qu'un aidant non professionnel.

### ARRIERE PLAN DE L'INVENTION

On connaît du document JP2006271821 une borne d'assistance reliée à des capteurs indiquant l'état de fonctionnement d'un respirateur connecté à une personne à surveiller. En cas de disfonctionnement du respirateur, le respirateur émet un signal immédiatement détecté par les capteurs et transmis à la borne d'assistance. La borne d'assistance transmet le signal à un dispositif lumineux situé par exemple dans un couloir et transitant par un téléphone portable et une plateforme de télémédecine. Le dispositif indique également le numéro de chambre, de lit, le nom de la personne à surveiller et le type de disfonctionnement du respirateur.

Ce type de dispositif assure, néanmoins, un niveau de sécurité relatif pour certains types d'utilisateurs. Par exemple, les personnes atteintes de la maladie d'ondine, se caractérisant par un disfonctionnement du système nerveux autonome, sont susceptibles, lors d'une phase de sommeil profond, de se trouver en hypoventilation entraînant une augmentation du taux de dioxyde de carbone dans le sang. Le maintien des fonctions vitales primaires de ces personnes, telle qu'une respiration régulière et suffisante, nécessite donc l'utilisation d'un respirateur mais aussi une surveillance permanente. Pour des raisons pratiques et économiques, les personnes à surveiller sont soignées à domicile. Dans un environnement non médical, tel qu'un domicile, la borne d'assistance décrite par le document JP2006271821 ne permet pas une intervention rapide sur la personne à surveiller ou sur le respirateur. En cas d'arrêt respiratoire ou d'un disfonctionnement du respirateur, la personne à surveiller ne peut pas intervenir elle-même sur le problème à résoudre puisque cette dernière est souvent incapable de se réveiller et ne se rend pas compte qu'un signal est émis pour signaler un dysfonctionnement.

De nombreux dispositifs du même type, connus de l'art antérieur, comportent une borne d'assistance incluse dans le dispositif médical. Ainsi, la borne d'assistance ne peut pas s'adapter à des dispositifs médicaux, tel qu'un respirateur, déjà existants.

Il est connu du document WO 2009/153535 une borne d'assistance permettant la surveillance à distance d'une personne reliée à un dispositif médical d'assistance et/ou de surveillance pouvant s'adapter à des dispositifs médicaux déjà existants dans des hôpitaux. Toutefois, une telle borne se contente de transmettre un signal d'alarme à un dispositif distant situé par exemple dans un couloir et transitant par un téléphone portable et une plateforme de télémédecine en indiquant notamment le numéro de chambre d'où provient le signal. Une telle borne s'avère donc ne pas pouvoir être utilisée partout car la présence d'un tiers est nécessaire pour intervenir sur le dispositif lorsque celui-ci émet un signal.

Le document US2006/0017558A1 divulgue aussi une borne d'assistance similaire.

### OBJET DE L'INVENTION

Le but de l'invention est donc de proposer une borne d'assistance permettant de s'adapter à tout type de dispositifs médicaux existants aptes à émettre au moins un signal qui obvie au moins en partie aux problèmes précités.

### RESUME DE L'INVENTION

A cet effet, l'invention a pour objet une borne d'assistance permettant la surveillance à distance d'une personne reliée à un dispositif médical d'assistance et/ou de surveillance, la borne d'assistance comportant un boîtier et au moins un terminal distant, le boîtier comportant :
- des moyens de réception pour recevoir un signal en provenance du dispositif médical;
- des moyens de conditionnement pour conditionner le signal reçu par les moyens de réception du boîtier;
- des moyens d'enregistrement pour, dans une phase d'apprentissage préalable à une phase d'exploitation de la borne, enregistrer une gamme de signaux qui sont émis par le dispositif médical, et qui sont reçus par les moyens de réception du boîtier et qui sont conditionnés par les moyens de conditionnement;
- des moyens de comparaison pour, lors de la phase d'exploitation de la borne, comparer un signal émis par le dispositif médical, reçu par les moyens de réception et conditionné par les moyens de conditionnement, avec les signaux préalablement enregistrés;
- des moyens de transmission pour, lors de la phase d'exploitation, transmettre le signal, si celui-ci correspond à un signal préalablement enregistré, au terminal distant;
le terminal comportant des moyens de restitution du signal à au moins une personne pour l'avertir de l'émission par le dispositif dudit signal.

Selon l'invention, les moyens de restitution du signal comportent des moyens d'émission d'au moins un stimulus nociceptif à la personne reliée au dispositif médical d'assistance et/ou de surveillance, lesdits moyens de restitution étant agencés pour contrôler les moyens d'émission de sorte que lesdits moyens d'émission augmentent progressivement une intensité du stimulus nociceptif.

Les différents moyens de réception, de conditionnement, d'enregistrement, de comparaison et de transmission contribuent à ce que la borne d'assistance puisse s'adapter à tout type de dispositif médical. Selon que le signal émis par le dispositif médical soit audio, vidéo, visuel, électrique... les moyens de réception du boîtier comportent des récepteurs assurant la réception d'une gamme de signaux émis par le dispositif médical.

De façon avantageuse, en menant une nouvelle phase d'apprentissage, la borne d'assistance est facilement re-programmable en cas de changement du dispositif médical associé.

Par ailleurs, la personne avertie de l'émission dudit signal est la personne reliée au dispositif médical d'assistance et/ou de surveillance de sorte que la personne à surveiller peut intervenir elle-même sur son propre état clinique ou sur le dispositif médical défectueux. La borne d'assistance selon l'invention est ainsi utilisable aussi bien pour une surveillance à domicile que dans un hôpital, la personne reliée au dispositif médical étant autonome.

### BREVE DESCRIPTION DES DESSINS

L'invention sera mieux comprise à la lumière de la description qui suit d'un mode de réalisation particulier de l'invention en relation avec les figures ci-jointes parmi lesquelles :
- la figure 1 est une vue d'une personne à surveiller et d'une borne d'assistance selon l'invention ;
- la figure 2 est un schéma fonctionnel de la borne de la figure 1.

### DESCRIPTION DETAILLEE DE L'INVENTION

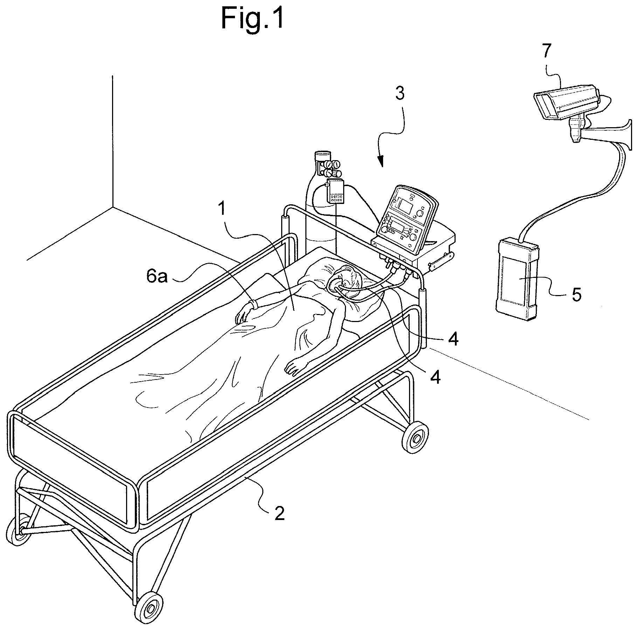

La figure 1 représente une personne à surveiller 1, se trouvant par exemple à son domicile, allongée dans un lit 2. La personne à surveiller 1 est reliée à un dispositif médical d'assistance et/ou de surveillance, par exemple ici un respirateur 3, permettant d'assurer les fonctions respiratoires de la personne à surveiller 1 en délivrant une ventilation artificielle. La pièce dans laquelle se trouve la personne à surveiller 1 comprend également un boîtier 5 disposé à proximité du respirateur 3, apte à capter des signaux, les transformer, les stocker, les comparer et les transmettre comme expliqué plus loin. La pièce comprend aussi au moins un terminal. Ici, le terminal comporte un bracelet 6a, fixé par exemple au poignet de la personne à surveiller 1. Le boîtier 5 et le terminal 6a constituent une borne d'assistance selon l'invention. D'autres terminaux peuvent être agencés dans ou à l'extérieur du domicile.

Dans le mode de réalisation présenté, une caméra 7 est connectée au boîtier 5 et transmet des données audio et vidéo via le boîtier 5.

Outre la fonction de ventilation artificielle, le respirateur 3 assure également une surveillance permanente de sa propre efficacité en contrôlant, d'une part, le volume gazeux délivré à la personne à surveiller 1 en le comparant à un volume prescrit par des réglages prédéfinis, et en contrôlant, d'autre part, la pression qui règne dans des conduits 4 reliant le respirateur 3 et la personne à surveiller 1. Lorsque le volume délivré à la personne à surveiller 1 est insuffisant, ou lorsque la pression dans les conduits 4 est trop haute ou trop basse (fuite, bouchon, débranchement, arrêt respiratoire ...), le respirateur 3 émet un signal d'alerte, par exemple un signal sonore.

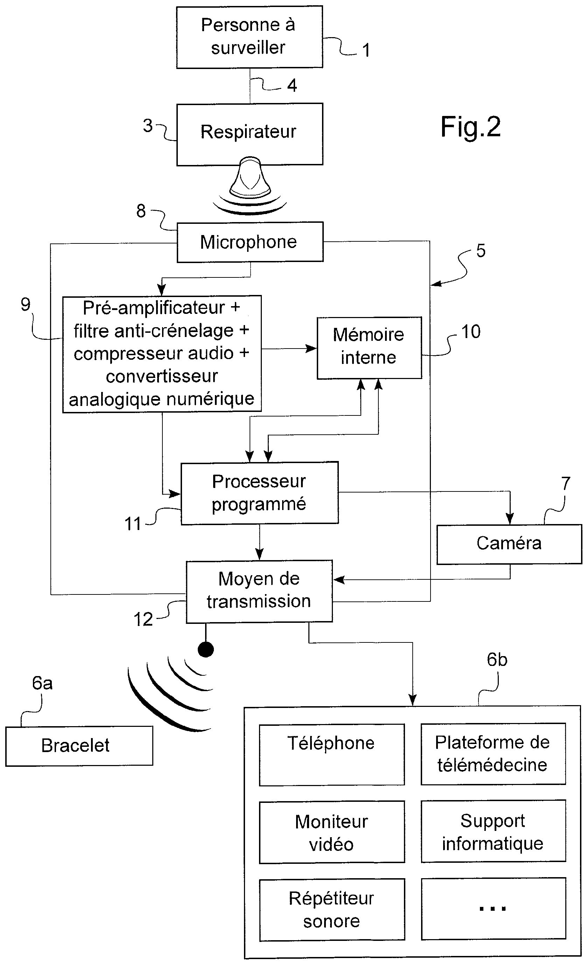

La figure 2 représente plus particulièrement le fonctionnement de la borne d'assistance de l'invention.

Dans le mode de réalisation présenté, le boîtier 5 comprend :
- des moyens de réception, tels qu'un microphone 8, pour recevoir le ou les signaux audio émis par le respirateur 3 ;
- des moyens de conditionnement 9 pour conditionner le ou les signaux reçus ;
- des moyens d'enregistrement, telle qu'une mémoire interne 10, pour enregistrer les signaux conditionnés lors d'une phase d'apprentissage ;
- des moyens de comparaison d'un signal reçu avec les signaux enregistrés, ici un processeur 11 programmé au moyen d'un logiciel effectuant la comparaison des signaux, et ;
- des moyens de transmission 12 des signaux si ceux-ci sont reconnus par les moyens de comparaison comme étant émis par le respirateur.

De manière générale, le ou les signaux sonores émis par le respirateur 3 ont des fréquences comprises entre 400 Hz et 4 KHz. L'un des avantages de la borne d'assistance est de pouvoir s'adapter à différents types de dispositifs médicaux. En conséquence, afin de ne pas se situer aux limites de détection du ou des signaux émis par le respirateur 3, le microphone 8 est capable de capter des signaux compris entre 200 Hz et 10 KHz pour une pression acoustique supérieure à 40 dBA.

Avant l'utilisation de la borne d'assistance, le boîtier 5 doit être paramétré de sorte que le ou les signaux audio émis par le respirateur 3 puissent être reconnus par le boîtier 5. La phase d'apprentissage, préalable à une phase d'exploitation de la borne, consiste à enregistrer la gamme de signaux émis par le respirateur 3. Pour cela, chacun des signaux audio émis par le respirateur 3 sont captés par le microphone 8 pour ensuite être conditionnés par les moyens de conditionnement 9 et enfin stockés dans la mémoire interne 10 du boîtier 5.

En cas de changement du dispositif médical d'assistance et/ou de surveillance associé à la borne d'assistance, il suffira ainsi de réinitialiser la phase d'apprentissage pour paramétrer de nouveau le boîtier 5.

Dans le mode de réalisation présenté, les moyens de conditionnement 9 comprennent un préamplificateur, agissant comme un passe bande, un filtre anti-crénelage, assurant qu'aucun des signaux réceptionnés n'ait une fréquence supérieure à 10 KHz, un compresseur audio et un convertisseur analogique numérique pour que les signaux puissent être stockés dans la mémoire interne 10 du boîtier 5.

Une fois la phase d'apprentissage réalisée, la borne d'assistance peut fonctionner. Ainsi, en cas de déclenchement d'un signal audio émis par le respirateur 3, le microphone 8 capte le signal qui est ensuite conditionné, puis comparé aux signaux préalablement enregistrés à l'aide du processeur 11. Si le signal conditionné correspond à l'un des signaux préalablement enregistrés, les moyens de transmission 12 transmettent le signal au bracelet 6a.

Ici le bracelet 6a, au contact de la personne à surveiller 1, reçoit le signal transmis par transmission sans fil. Le bracelet 6a comporte des moyens de restitution du signal à la personne à surveiller 1 pour l'avertir de l'émission du signal par le dispositif médical 3. Selon l'invention, les moyens de restitution du signal comportent des moyens d'émission d'au moins un stimulus nociceptif à la personne à surveiller 1.

Ici, les moyens d'émission d'un stimulus nociceptif comportent un dispositif nociceptif monté sur le bracelet 6a.

Selon un mode de réalisation privilégié, le dispositif nociceptif comporte des moyens de génération d'impulsions électriques à la personne à surveiller 1. Dans le cas de la maladie d'ondine, les impulsions électriques suffisent normalement à réveiller la personne à surveiller même si celle-ci est dans une phase de sommeil profond. La personne est ainsi autonome.

De préférence, les moyens de restitution du signal sont agencés pour contrôler le dispositif nociceptif de sorte que ledit dispositif nociceptif augmente progressivement les impulsions électriques transmises, de préférence jusqu'à désactivation du dispositif nociceptif. De préférence, les impulsions électriques ne sont interrompues que si le signal émis par le respirateur 3 est désactivé.

De façon privilégiée, les moyens de restitution du signal comportent en outre un dispositif vibratoire qui est également monté sur le bracelet 6a.

De la sorte, il est ainsi possible d'émettre dans un premier temps des vibrations à la personne à surveiller 1 avant de lui émettre des impulsions électriques si les vibrations ne sont pas suffisantes pour la faire réagir.

Selon un mode particulier de réalisation, les moyens de restitution du signal sont agencés pour contrôler les dispositifs vibratoire et nociceptif de sorte que des vibrations soient émises à l'aide du dispositif vibratoire puis que des impulsions électriques soient émises à l'aide du dispositif nociceptif, en remplacement des vibrations, notamment si le dispositif vibratoire n'est pas désactivé au bout d'un intervalle de temps déterminé à partir de l'émission d'une première vibration. De préférence, les vibrations ne sont interrompues que si le signal émis par le respirateur 3 est désactivé.

Ainsi, si la personne à surveiller 1 est réveillée, les vibrations suffisent à l'avertir que le dispositif médical 3 émet un signal. Si la personne à surveiller 1 est endormie et que les vibrations ne suffisent pas à la réveiller, les impulsions électriques devront normalement réveiller la personne à surveiller même si celle-ci est dans une phase de sommeil profond.

Selon un mode de réalisation privilégié, la borne 5 comporte un deuxième terminal 6b comportant un moniteur vidéo pour avertir une tierce personne de l'émission du signal par le dispositif médical. Le moniteur vidéo reçoit au moins des données vidéo de la caméra 7 en cas d'émission du signal par le dispositif médical 3.

Ainsi, en cas de déclenchement d'un signal audio en provenance du respirateur 3, la personne à surveiller 1 est en mesure de se réveiller et d'intervenir sur son état physique ou sur le respirateur. Grâce au deuxième terminal 6b, la tierce personne est également avertie de l'anomalie.

De façon avantageuse, en cas d'émission du signal par le dispositif médical 3, la tierce personne peut dans un premier temps visualiser la personne à surveiller 1 via le moniteur vidéo et constater s'il convient d'intervenir ou non. Par exemple, si la personne à surveiller se réveille d'elle-même et agit sur le dispositif médical à surveiller, la tierce personne peut facilement s'assurer que son intervention n'est pas nécessaire.

Selon un mode réalisation privilégié, les moyens de transmission 12 sont agencés pour transmettre le signal au premier terminal 6a puis au deuxième terminal 6b en remplacement ou en complément de la transmission du signal au premier terminal. De préférence, les moyens de transmission 12 sont agencés de sorte que :
- des vibrations soient émises à l'aide du dispositif vibratoire ;
- puis que des impulsions électriques soient émises à l'aide du dispositif nociceptif, en remplacement ou en complément des vibrations, si le dispositif vibratoire n'est pas désactivé au bout d'un intervalle de temps déterminé à partir de l'émission d'une première vibration ;
- et enfin que des données vidéos soient émises au moniteur vidéo du deuxième terminal 6b si le dispositif nociceptif n'est pas désactivé au bout d'un intervalle de temps déterminé à partir de l'émission de la première impulsion électrique.

Selon un aspect de particulier de l'invention et pour des raisons de sécurité, le signal restitué par les terminaux 6a, 6b ne peut être désactivé qu'en réponse à une détection par le respirateur 3 d'un retour à la normale du respirateur 3 selon les paramètres du respirateur 3 prédéfinis. De préférence, le signal restitué n'est interrompu que si le signal émis par le respirateur 3 est désactivé, ce qui est le signe que quelqu'un à traité le problème signalé par l'émission du signal. De préférence, les moyens de restitution du signal du premier terminal (6a) et du deuxième terminal (6b) sont agencés pour désactiver le signal restitué en réponse uniquement :
- à une détection par le dispositif médical 3 d'une amélioration de l'état clinique de la personne à surveiller 1 et l'émission par le dispositif médical 3 d'un signal correspondant, ou
- à la réparation d'un disfonctionnement du dispositif médical 3 et l'émission par le dispositif médical 3 d'un signal correspondant.

Bien entendu, l'invention n'est pas limitée au mode de réalisation décrit ci-dessus et est susceptible de variantes qui apparaîtront à l'homme de métier sans sortir du cadre de l'invention tel que défini par les revendications.

Bien entendu, la borne d'assistance est utilisable avec tout dispositif médical d'assistance et/ou de surveillance, et pas seulement un respirateur.

La borne d'assistance selon l'invention pourra également remplir d'autres fonctions que celles de surveiller un patient et d'avertir le patient et/ou un tiers en cas de problème. Par exemple, la borne d'assistance pourra comporter des moyens pour mémoriser les signaux qu'elle reçoit du dispositif médical. Ces signaux pourront ainsi être récupérés pour être analysés ultérieurement. Par exemple, dans le cas de la maladie d'ondine, les signaux mémorisés durant la nuit pourront être analysés dans la journée.

Bien que dans l'exemple illustré, le signal émis par le dispositif médical soit du type sonore et par conséquent que le moyen de réception 8 du boîtier 5 soit un microphone, le dispositif médical pourra émettre d'autres types de signaux, comme par exemple visuel, électrique ou autres, les moyens de réception 8 du boîtier 5 seront adaptés à recevoir de tels signaux.

Les moyens de transmission 12 pourront être de tout type. De préférence, les moyens de transmission 12 dépendront de l'emplacement du terminal associé. Si le terminal est proche ou au contact de la personne à surveiller, on utilisera de préférence un mode de transmission sans fil, par exemple de type bluetooth. Si le terminal est éloigné de la personne à surveiller, on utilisera par exemple le réseau internet. On pourra également utiliser un réseau téléphonique.

Par ailleurs, la borne 5 pourra communiquer avec d'autres terminaux qu'un moniteur vidéo que ceux décrits, comme un téléphone, un répétiteur sonore, une plateforme de télémédecine, tout type de support informatique, tel qu'un ordinateur. De préférence, l'état médical du patient permettra de déterminer avec combien et quels terminaux la borne 5 pourra communiquer. Par exemple, pour un patient avec un handicap respiratoire léger, la borne 5 pourra être uniquement en communication avec le bracelet 6a. Pour une personne ayant des problèmes moteurs et un handicap respiratoire lourd, il serait préférable que la borne 5 soit en outre en communication avec un deuxième terminal par exemple directement relié à un service d'urgence comme le SAMU.

Bien qu'ici on ait dit que le bracelet 6a comportait un dispositif vibratoire et un dispositif nociceptif, le bracelet 6a ne pourra comporter que le dispositif nociceptif.

De la même façon, le bracelet 6a pourra comporter tout autre moyen de restitution de signal que ceux décrits en combinaison avec les moyens d'émission d'un stimulus nociceptif. Par exemple, le bracelet 6a pourra comporter en outre un dispositif sonore qui émet une alarme sonore jusqu'à désactivation du signal émis par le dispositif médical 3 en combinaison de l'émission de stimulus nociceptif par le dispositif nociceptif.

Ainsi, le bracelet 6a pourra restituer le signal par chaque dispositif qu'il comporte de façon successive ou simultanée. Par exemple, le bracelet 6a sera adapté dans un premier temps à restituer le signal par l'un des dispositifs qu'il comporte puis, si le signal émis par le dispositif médical n'est pas désactivé, à restituer le signal par un autre des dispositifs. Le bracelet 6a pourra également être adapté à restituer le signal par les deux dispositifs en même temps si le signal émis par le dispositif médical n'est pas désactivé.

Selon un mode particulier de réalisation, les moyens de restitution du signal sont agencés pour contrôler les dispositifs vibratoire et nociceptif de sorte que des vibrations soient émises à l'aide du dispositif vibratoire puis que des impulsions électriques soient émises à l'aide du dispositif nociceptif, en complément des vibrations, si le dispositif vibratoire n'est pas désactivé au bout d'un intervalle de temps déterminé à partir de l'émission d'une première vibration.

## Revendications

1. Borne d'assistance (5, 6a, 6b) permettant la surveillance à distance d'une personne (1) reliée à un dispositif médical (3) d'assistance et/ou de surveillance, la borne d'assistance comportant un boîtier et au moins un terminal distant, le boîtier (5) comportant :
- des moyens de réception (8) pour recevoir un signal en provenance du dispositif médical (3) ;
- des moyens de conditionnement (9) pour conditionner le signal reçu par les moyens de réception (8) du boîtier 5) ;
- des moyens d'enregistrement (10) pour, dans une phase d'apprentissage préalable à une phase d'exploitation de la borne (5, 6a, 6b), enregistrer une gamme de signaux qui sont émis par le dispositif médical (3), et qui sont reçus par les moyens de réception (8) du boîtier (5) et qui sont conditionnés par les moyens de conditionnement (9) ;
- des moyens de comparaison (11) pour, lors de la phase d'exploitation de la borne (5, 6a, 6b), comparer un signal émis par le dispositif médical (3), reçu par les moyens de réception (8) et conditionné par les moyens de conditionnement (9), avec les signaux préalablement enregistrés ;
- des moyens de transmission (12) pour, lors de la phase d'exploitation, transmettre le signal, si celui-ci correspond à un signal préalablement enregistré, au terminal distant (6a, 6b) ;
le terminal (6a, 6b) comportant des moyens de restitution du signal à au moins une personne pour l'avertir de l'émission par le dispositif dudit signal ;
la borne d'assistance étant **caractérisée en ce que** les moyens de restitution du signal comportent des moyens d'émission d'au moins un stimulus nociceptif à la personne reliée au dispositif médical d'assistance et/ou de surveillance, lesdits moyens de restitution étant agencés pour contrôler les moyens d'émission de sorte que lesdits moyens d'émission augmentent progressivement une intensité du stimulus nociceptif.

2. Borne d'assistance selon la revendication 1, dans laquelle le terminal comporte un bracelet destiné à être porté par la personne reliée au dispositif médical d'assistance et/ou de surveillance, les moyens d'émission de stimulus nociceptif comportant un dispositif nociceptif monté sur le bracelet.

3. Borne d'assistance selon la revendication 2, dans laquelle le dispositif nociceptif comporte des moyens de génération d'impulsions électriques à la personne reliée au dispositif médical d'assistance et/ou de surveillance.

4. Borne d'assistance selon la revendication 2, dans laquelle les moyens de restitution du signal comportent en outre un dispositif vibratoire qui est monté sur le bracelet.

5. Borne d'assistance selon la revendication 4, dans lequel les moyens de restitution sont agencés pour contrôler les dispositifs nociceptif et vibratoire de sorte que des vibrations soient émises à l'aide du dispositif vibratoire puis que des impulsions électriques soient émises à l'aide du dispositif nociceptif, en remplacement ou en complément des vibrations.

6. Borne d'assistance selon la revendication 1, dans laquelle les moyens de réception (8) comprennent une caméra (7), les moyens de transmission (12) étant aptes à transmettre un signal vidéo généré par la caméra.

7. Borne d'assistance selon la revendication 6, comportant un deuxième terminal (6b) qui comprend un moniteur vidéo recevant le signal vidéo.

8. Borne d'assistance selon la revendication 7, dans laquelle les moyens de transmission (12) sont agencés pour transmettre le signal au premier terminal (6a) puis au deuxième terminal (6b) en remplacement ou en complément de la transmission du signal au premier terminal.

9. Borne d'assistance selon la revendication 1, dans lequel les moyens de restitution du signal du terminal (6a, 6b) distant sont agencés pour désactiver le signal restitué en réponse uniquement :
- à une détection par le dispositif médical (3) d'une amélioration de l'état clinique de la personne reliée au dispositif médical d'assistance et/ou de surveillance (1) et l'émission par le dispositif médical (3) d'un signal correspondant, ou
- à la réparation d'un disfonctionnement du dispositif médical (3) et l'émission par le dispositif médical (3) d'un signal correspondant.

## Patentansprüche

1. Unterstützungsendgerät (5, 6a, 6b), das die Fernüberwachung einer Person (1) ermöglicht, die mit einer medizinischen Unterstützungs- und/oder Überwachungsvorrichtung (3) verbunden ist, wobei das Unterstützungsendgerät ein Gehäuse und mindestens einen Fernterminal umfasst, wobei das Gehäuse (5) umfasst:
- Empfangsmittel (8), um ein Signal zu empfangen, das aus der medizinischen Vorrichtung (3) stammt;
- Konditionierungsmittel (9), um das Signal zu konditionieren, das von den Empfangsmitteln (8) des Gehäuses (5) empfangen wurde;
- Speichermittel (10), um in einer Lernphase vor einer Nutzungsphase des Endgeräts (5, 6a, 6b) einen Bereich von Signalen zu speichern, die von der medizinischen Vorrichtung (3) gesendet und von den Empfangsmitteln (8) des Gehäuses (5) empfangen und von den Konditionierungsmitteln (9) konditioniert werden;
- Vergleichsmittel (11), um während der Nutzungsphase des Endgeräts (5, 6a, 6b) ein Signal, das von der medizinischen Vorrichtung (3) gesendet, von den Empfangsmitteln (8) empfangen und von den Konditionierungsmitteln (9) konditioniert wurde, mit den zuvor gespeicherten Signalen zu vergleichen;
- Übertragungsmittel (12), um während der Nutzungsphase das Signal zu dem Fernterminal (6a, 6b) zu übertragen, wenn dieses Signal einem zuvor gespeicherten Signal entspricht;
wobei der Terminal (6a, 6b) Wiedergabemittel zur Wiedergabe des Signals an mindestens eine Person umfasst, um sie über das Senden des Signals durch die Vorrichtung zu benachrichtigen;
wobei das Unterstützungsendgerät **dadurch gekennzeichnet ist, dass** die Wiedergabemittel zur Wiedergabe des Signals Sendemittel zum Senden mindestens eines nozizeptiven Stimulus an die Person umfassen, die mit der medizinischen Unterstützungs- und/oder Überwachungsvorrichtung verbunden ist, wobei die genannten Wiedergabemittel ausgebildet sind, um die Sendemittel derart zu steuern, dass die genannten Sendemittel eine Intensität des nozizeptiven Stimulus nach und nach erhöhen.

2. Unterstützungsendgerät nach Anspruch 1, bei dem der Terminal ein Armband umfasst, das dazu bestimmt ist, von der Person getragen zu werden, die mit der medizinischen Unterstützungs- und/oder Überwachungsvorrichtung verbunden ist, wobei die Sendemittel zum Senden des nozizeptiven Stimulus eine an dem Armband angebrachte nozizeptive Vorrichtung umfassen.

3. Unterstützungsendgerät nach Anspruch 2, bei dem die nozizeptive Vorrichtung Mittel zum Erzeugen elektrischer Impulse an die Person umfasst, die mit der medizinischen Unterstützungs- und/oder Überwachungsvorrichtung verbunden ist.

4. Unterstützungsendgerät nach Anspruch 2, bei dem die Signalwiedergabemittel ferner eine Vibrationsvorrichtung umfassen, die an dem Armband angebracht ist.

5. Unterstützungsendgerät nach Anspruch 4, bei dem die Wiedergabemittel ausgebildet sind, um die nozizeptive Vorrichtung und die Vibrationsvorrichtung derart zu steuern, dass Vibrationen mit Hilfe der Vibrationsvorrichtung abgegeben werden und dann elektrische Impulse mit Hilfe der nozizeptiven Vorrichtung abgegeben werden, anstelle oder zusätzlich zu den Vibrationen.

6. Unterstützungsendgerät nach Anspruch 1, bei dem die Empfangsmittel (8) eine Kamera (7) umfassen, wobei die Übertragungsmittel (12) dazu geeignet sind, ein von der Kamera erzeugtes Videosignal zu übertragen.

7. Unterstützungsendgerät nach Anspruch 6, umfassend einen zweiten Terminal (6b), der einen Videomonitor umfasst, der das Videosignal empfängt.

8. Unterstützungsendgerät nach Anspruch 7, bei dem die Übertragungsmittel (12) ausgebildet sind, um das Signal zu dem ersten Terminal (6a) und dann zu dem zweiten Terminal (6b) zu übertragen, anstelle oder zusätzlich zur Übertagung des Signals zum ersten Terminal.

9. Unterstützungsendgerät nach Anspruch 1, bei dem die Wiedergabemittel zur Wiedergabe des Signals des Fernterminals (6a, 6b) ausgebildet sind, um das wiedergegebene Signal nur zu deaktivieren in Antwort auf:
- eine Erfassung durch die medizinische Vorrichtung (3) einer Verbesserung des klinischen Zustandes der Person, die mit der medizinischen Unterstützungs- und/oder Überwachungsvorrichtung (1) verbunden ist, und die Aussendung eines entsprechenden Signals durch die medizinische Vorrichtung (3), oder
- die Behebung einer Fehlfunktion der medizinischen Vorrichtung (3) und die Aussendung eines entsprechenden Signals durch die medizinische Vorrichtung (3).

## Claims

1. An assistance terminal (5, 6a, 6b) for remotely monitoring a person (1) connected to a medical assistance and/or monitoring device (3), the assistance terminal comprising a housing and at least one remote terminal, the housing (5) comprising:
- reception means (8) for receiving a signal from the medical device (3);
- conditioning means (9) for conditioning the signal received by the reception means (8) of the housing (5);
- storage means (10) for, in a learning phase prior to a phase of use of the terminal (5, 6a, 6b), storing a range of signals which are sent by the medical device (3), and which are received by the reception means (8) of the housing (5) and which are conditioned by the conditioning means (9);
- comparison means (11) for, during the phase of use of the terminal (5, 6a, 6b), comparing a signal sent by the medical device (3), received by the reception means (8) and conditioned by the conditioning means (9), with the signals previously stored;
- transmission means (12) for, during the phase of use, transmitting the signal, if the latter corresponds to a signal previously stored, to the remote terminal (6a, 6b); the terminal (6a, 6b) comprising means for playing back the signal to at least one person to notify him or her of the sending of said signal by the device;
the assistance terminal being **characterized in that** the signal playback means comprise means for sending at least one nociceptive stimulus to the person connected to the medical assistance and/or monitoring device in which the playback means are arranged to control the sending means so that said sending means progressively increase an intensity of the nociceptive stimulus.

2. The assistance terminal as claimed in claim 1, in which the terminal comprises a bracelet intended to be worn by the person connected to the medical assistance and/or monitoring device, the means for sending the nociceptive stimulus comprising a nociceptive device mounted on the bracelet.

3. The assistance terminal as claimed in claim 2, in which the nociceptive device comprises means for generating electrical pulses to the person connected to the medical assistance and/or monitoring device.

4. The assistance terminal as claimed in claim 2, in which the means for playing back the signal also comprise a vibratory device which is mounted on the bracelet.

5. The assistance terminal as claimed in claim 4, in which the playback means are arranged to control the nociceptive and vibratory devices so that vibrations are sent using the vibratory device and then electrical pulses are sent using the nociceptive device, instead of or in addition to the vibrations.

6. The assistance terminal as claimed in claim 1, in which the reception means (8) comprise a camera (7), the transmission means (12) being capable of transmitting a video signal generated by the camera.

7. The assistance terminal as claimed in claim 6, comprising a second terminal (6b) which comprises a video monitor receiving the video signal.

8. The assistance terminal as claimed in claim 7, in which the transmission means (12) are arranged to transmit the signal to the first terminal (6a) then to the second terminal (6b) instead of or in addition to the transmission of the signal to the first terminal.

9. The assistance terminal as claimed in claim 1, in which the signal playback means of the remote terminal (6a, 6b) are arranged to deactivate the signal played back in response only:
- to a detection by the medical device (3) of an improvement in the clinical state of the person (1) connected to the medical assistance and/or monitoring device and the sending by the medical device (3) of a corresponding signal, or
- to the repair of a malfunction of the medical device (3) and the sending by the medical device (3) of a corresponding signal.
